# EUROPEAN PATENT APPLICATION

(11) **EP 4 148 119 A1**
(43) Date of publication of application: **15.03.2023**
(21) Application number: 21796751.2
(22) Date of filing: 23.04.2021
(51) Int. Cl.: C12N 1/20, A23L 33/135, A23L 33/10, C12R 1/46

(54) **NOVEL LACTIC ACID BACTERIA HAVING EXCELLENT IMMUNE FUNCTION ENHANCEMENT EFFECT, AND FOOD COMPOSITION, HEALTH FUNCTIONL FOOD COMPOSITION AND PROBIOTICS COMPRISING SAME**

(30) Priority: 29.04.2020 KR 20200052742
(71) Applicant: Green Cross Wellbeing Corporation, Seoul 07335 (KR)
(72) Inventor: JANG, Minjung, Seoul 07335 (KR); HONG, Gyeongeun, Seoul 07335 (KR); PARK, Gideok, Seoul 07335 (KR); KIM, Jaeyoung, Seoul 07335 (KR); KIM, Hyunsu, Seoul 07335 (KR); HWANG, Yongpil, Jinju-si Gyeongsangnam-do 52849 (KR)
(74) Representative: dompatent von Kreisler Selting Werner - Partnerschaft von Patent- und Rechtsanwälten mbB
(86) International application number: PCT/KR2021/005195
(87) International publication number: WO 2021/221398

(57) **Abstract**

The present disclosure provides a novel *Lactococcus lactis* GCWB1176 strain deposited under Accession No. KCCM12687P, having an excellent immune function enhancing effect.

In addition, the present disclosure provides a food composition, a health functional food composition and probiotics having an excellent immune function enhancing effect, comprising the novel strain.

## Description

### [Technical Field]

This application claims the benefit of Korean Patent Application No. 10-2020-0052742 filed on April 29, 2020, with the Korean Intellectual Property Office, the disclosure of which is herein incorporated by reference in its entirety.

The present disclosure relates to novel lactic acid bacteria having an excellent immune function enhancing effect, and a food composition, a health functional food composition and probiotics comprising the same.

### [Background Art]

Living microorganisms that have a beneficial effect on the health of the host by improving the intestinal microbial environment of the host in the gastrointestinal tract of animals including humans are collectively called probiotics.

Lactic acid bacteria, which are a type of probiotics, play a role in making important nutrients by decomposing fibers and complex proteins while having a symbiotic relation in the digestive system of the human body. Lactic acid bacteria are bacteria that decompose carbohydrates and produce lactic acid by using them, and are facultative anaerobes or obligate anaerobes that proliferate well in low oxygen conditions. Lactic acid bacteria can be broadly divided into the following five genera: *Streptococcus, Lactobacillus, Leuconostoc, Bifidobacterium,* and *Pediococcus.*

The microorganism of the genus *Streptococcus,* which is a streptococcus, is a bacterium that performs homozygous fermentation, and is known to inhibit putrefactive bacteria and pathogens by fermenting milk to produce lactic acid. The microorganism of the genus *Lactobacillus* is a lactic acid bacillus that performs homozygous or heterozygous fermentation and can be commonly seen in the fermentation process of dairy products or vegetables, and the microorganism of the genus *Leuconostoc,* which is a diplococcus, performs heterozygous fermentation and is mainly involved in the fermentation of vegetables. In addition, the microorganism of the genus *Bifidobacterium* is an obligate anaerobe that cannot grow in the presence of oxygen, and ferments sugars to produce L(+)-type lactic acid that can be metabolized usefully in infants. Finally, the microorganism of the genus *Pediococcus* is a bacterium that performs homozygous fermentation, having the form of tetrad, and is present in kimchi or pickled foods, and is involved in the fermentation of meat such as sausages.

Recently, as interest in immunity enhancement has increased, various studies have been made on immune enhancement methods using lactic acid bacteria. Since lactic acid bacteria are safe for the human body and provide various beneficial effects to the human body in addition to immunity enhancement, it is expected to be of great help to human health if lactic acid bacteria that are also effective for immune enhancement can be obtained.

However, lactic acid bacteria, which have been known to have immune-enhancing effects so far, did not have sufficient effects on immune enhancement, so there was a disadvantage in that they are insufficient to be used for immune-enhancing purposes.

### [Prior Art Document]

Patent Document 1. Korean Patent Publication No. 10-2016-0082863

### [Disclosure]

### [Technical Problem]

It is an object of the present disclosure to provide a novel *Lactococcus lactis* GCWB1176 strain that increases the amount of nitric oxide (NO) produced, increases cytokine (TNF-alpha, IFN-gamma, IL-1beta, IL-10, IL-12, and the like) secretion, increases NK cell activity and cell proliferation rate, and increases the phagocytic activity of macrophages.

It is another object of the present disclosure to provide a food composition, a health functional food composition and probiotics comprising the *Lactococcus lactis* GCWB1176 strain having an excellent immune-enhancing effect as described above.

### [Technical Solution]

In order to achieve the above objects,
the present disclosure
provides a *Lactococcus lactis* GCWB1176 strain deposited under Accession No. KCCM12687P.

In addition, the present disclosure provides a food composition for enhancing immune function, comprising a *Lactococcus lactis* GCWB1176 strain deposited under Accession No. KCCM12687P, one selected from a spray-dried product, a freeze-dried product, a vacuum-dried product, a drum-dried product or a crushed product of the strain, or any one of a culture of the strain, a concentrate, a paste, and a dilution of the culture.

In addition, the present disclosure provides a health functional food composition for enhancing immune function, comprising a *Lactococcus lactis* GCWB1176 strain deposited under Accession No. KCCM12687P, one selected from a spray-dried product, a freeze-dried product, a vacuum-dried product, a drum-dried product or a crushed product of the strain, or any one of a culture of the strain, a concentrate, a paste, and a dilution of the culture.

In addition, the present disclosure
provides probiotics comprising a *Lactococcus lactis* GCWB1176 strain deposited under Accession No. KCCM12687P.

### [Advantageous Effects]

The *Lactococcus lactis* GCWB1176 strain, which is the novel strain of the present disclosure, provides the effects of significantly increasing the amount of nitric oxide (NO) produced, remarkably increasing cytokine (TNF-alpha, IFN-gamma, IL-1beta, IL-10, IL-12, and the like) secretion, significantly increasing NK cell activity and cell proliferation rate, and increasing the phagocytic activity of macrophages. Accordingly, the *Lactococcus lactis* GCWB1176 strain of the present disclosure provides an excellent immune function enhancing effect.

In addition, the food composition, health functional food composition and probiotics of the present disclosure provide an excellent immune function enhancing effect by comprising the *Lactococcus lactis* GCWB1176 strain.

### [Description of Drawings]

FIG. 1 is a graph showing the measurement of the amount of nitric oxide (NO) produced by five strain samples in the macrophage line (Experimental Example 1-2);
FIGS. 2 and 3 are graphs showing the measurement of phagocytic activity by five strain samples in the macrophage line (Experimental Example 1-4);
FIG. 4 is a graph showing the measurement of the splenocyte proliferation rate of ICR mouse by five strain samples (Experimental Example 2-1);
FIG. 5 is a graph showing the measurement of the amount of nitric oxide (NO) produced by five strain samples in splenocyte line of ICR mouse (Experimental Example 2-2);
FIG. 6 is a graph showing the measurement of changes in the body weight, spleen weight and thymus weight of the mouse according to administration of the sample *Lactococcus lactis* GCWB1176 strain in the immunosuppressive agent cyclophosphamide-induced immunosuppressed SPF ICR mouse model (Experimental Example 3-2);
FIG. 7 is a graph showing the measurement of the change in the cytokine secretion according to administration of the sample *Lactococcus lactis* GCWB1176 strain in the immunosuppressive agent cyclophosphamide-induced immunosuppressed SPF ICR mouse model (Experimental Example 3-3); and
FIG. 8 is a graph showing the measurement of changes in NK cell activity and cell proliferation rate according to administration of the sample *Lactococcus lactis* GCWB1176 strain in the immunosuppressive agent cyclophosphamide-induced immunosuppressed SPF ICR mouse model (Experimental Example 3-4).

### [Best Mode]

Hereinafter, the present disclosure will be described in more detail.

Unless defined otherwise, all technical terms used in the present disclosure have the same meaning as commonly understood by those skilled in the relevant field of the present disclosure. In addition, preferred methods or samples are described in the present specification, but similar or equivalent ones are also included in the scope of the present disclosure.

The present disclosure
relates to a *Lactococcus lactis* GCWB1176 strain deposited under Accession No. KCCM12687P.

The novel strain, *Lactococcus lactis* GCWB1176 was deposited with the Korean Culture Center of Microorganisms on March 31, 2020 under the name as described above. The accession number is KCCM12687P.

The strain may be isolated and identified from cheeses collected in each region. The present inventors have completed the present disclosure by confirming that various novel strains were isolated and identified from cheeses collected from each region, and among them, a *Lactococcus lactis* GCWB1176 strain provided a remarkably excellent immune function enhancing effect compared to conventional known lactic acid bacteria.

That is, the *Lactococcus lactis* GCWB1176 strain provides the effect of significantly increasing the amount of nitric oxide (NO) produced. In terms of immune response, immune cells are activated only when there is an initial inflammatory response, and immunity is thereby enhanced, and thus, in contrast to anti-inflammatory, the occurrence of NO rather plays a role in increasing immunity.

In addition, it provides the effects of increasing cytokine (TNF-alpha, IFN-gamma, IL-lbeta, IL-10, IL-12, and the like) secretion, and remarkably increasing NK cell activity and cell proliferation rate.

In addition, it provides the effect of increasing the phagocytic activity of macrophages.

In particular, the *Lactococcus lactis* GCWB1176 strain provides, *in vitro* and *in vivo,* provides the effect of very remarkably increasing cytokine (TNF-alpha, IFN-gamma, IL-lbeta, IL-10, IL-12, and the like) secretion and provides the effect of improving NK cell activity and cell proliferation rate.

The strain of the present disclosure may be used that has, but are not limited to, a live bacterial content of 1 × 10¹ to 1 × 10¹³ CFU/g.

In addition, the present disclosure
relates to a food composition for enhancing immune function, comprising a *Lactococcus lactis* GCWB1176 strain deposited under Accession No. KCCM12687P, one selected from a spray-dried product, a freeze-dried product, a vacuum-dried product, a drum-dried product or a crushed product of the strain, or any one of a culture of the strain, a concentrate, a paste, and a dilution of the culture.

In addition, the present disclosure
relates to a health functional food composition for enhancing immune function, comprising a *Lactococcus lactis* GCWB1176 strain deposited under Accession No. KCCM12687P, one selected from a spray-dried product, a freeze-dried product, a vacuum-dried product, a drum-dried product or a crushed product of the strain, or any one of a culture of the strain, a concentrate, a paste, and a dilution of the culture.

Immunity is a self-protective system that exists in the body, and is a process in which the human body recognizes, removes and metabolizes various substances or living things that invade from the outside as foreign substances against itself. It protects itself from damage caused by external stimuli or invasion of pathogenic microorganisms, but it may also damage its own tissues, like an inflammatory response. Immunity is the action of regulating changes in immune function to restore it to normal or reduce the width of the change, and is divided into immune function suppression or immune function enhancement.

In the present disclosure, the immune function enhancement refers to a function of enhancing the bioprotective ability by regulating immunity. Biomarkers that can confirm the functionality of the immune function enhancement include NK cells, cytokines, phagocytic activity, NO, iNOS, COX2, complement system, lymphocyte subpopulation ratio, immunoglobulin, cell viability, splenocyte proliferation, weight of tissues such as spleen and thymus, and the like.

The functionality of the health functional food composition for enhancing immune function of the present disclosure is confirmed by the functional markers in the Examples and Test Examples below.

In addition, the present disclosure
relates to a pharmaceutical composition for preventing and treating an immunodeficiency disease or an inflammatory disease, comprising a *Lactococcus lactis* GCWB1176 strain deposited under Accession No. KCCM12687P, one selected from a spray-dried product, a freeze-dried product, a vacuum-dried product, a drum-dried product or a crushed product of the strain, or any one of a culture of the strain, a concentrate, a paste, and a dilution of the culture.

In the pharmaceutical composition, the immunodeficiency diseases may include immunodeficiency, human immunodeficiency virus disease, intestinal infectious disease, specific infectious disease, bacterial infection, fungal infection, parasitic disease, allergic disease, fatigue, transient heat fatigue, malaise and fatigue, neurasthenia, and the like;
and the inflammatory diseases may include chronic and acute rhinitis, chronic and acute gastritis, enteritis, ulcerative gastritis, acute and chronic nephritis, acute and chronic hepatitis, chronic obstructive pulmonary disease, pulmonary fibrosis, irritable bowel syndrome, inflammatory pain, back pain, fibromyalgia, myofascial disease, gout, arthritis, rheumatoid arthritis, ankylosing spondylitis, Hodgkin's disease, pancreatitis, conjunctivitis, iritis, scleritis, uveitis, dermatitis, atopic dermatitis, eczema, tuberculous female pelvic inflammatory disease, inflammatory arthritis, inflammatory diseases of the central nervous system, inflammatory polyneuropathy, and the like.

In the present disclosure, the food composition or health functional food composition as described above may comprise ingredients that are commonly added during food preparation, in addition to the active ingredient, and, for example, may comprise proteins, carbohydrates, fats, nutrients, seasonings, sweeteners, and flavoring agents, but is not limited thereto. Examples of the carbohydrates include conventional sugars, such as monosaccharides, for example, glucose, fructose, and the like; disaccharides, for example, maltose, sucrose, oligosaccharide, and the like; and polysaccharides, for example, dextrin, cyclodextrin, and the like, and sugar alcohols such as xylitol, sorbitol, and erythritol. As the sweeteners, natural sweeteners (taumatin, stevia extracts, rebaudioside A, glycyrrhizin, and the like) and synthetic sweeteners (saccharin, aspartame, and the like) may be used. However, it is not limited to them, and any of the ingredients known in the art that do not impair the effects of the present disclosure may be used.

Examples of the food composition or health functional food composition may include, but are not limited to, patient nutrition, meat, cereals, caffeine drinks, general drinks, dairy products, chocolates, bread, snacks, confectionery, pizza, jelly, noodles, gums, ice cream, alcoholic beverages, alcohols, vitamin complexes, other health supplement foods, and the like. When prepared in the form of the food composition or health functional food composition as described above, it is preferable because it may be conveniently and easily taken to enhance immune function.

In the present disclosure, the health functional food composition and pharmaceutical composition may be prepared in the form of granules, lemonades, powders, syrups, liquids and solutions, extracts, elixirs, fluid extracts, suspensions, decoctions, infusions, tablets, spirits, capsules, troches, pills, or soft or hard gelatin capsules, but are not limited thereto.

The pharmaceutical composition as described above may further comprise a pharmaceutically acceptable carrier. The pharmaceutically acceptable carriers comprised in the pharmaceutical composition of the present disclosure are those conventionally used in the formulation and include, but are not limited to, lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methylcellulose, methylhydroxybenzoate, propylhydroxybenzoate, talc, magnesium stearate, mineral oil, and the like.

The pharmaceutical composition of the present disclosure may further comprise a lubricant, a wetting agent, a sweetener, a flavoring agent, an emulsifier, a suspending agent, a preservative, and the like, in addition to the ingredients as described above.

The pharmaceutical composition of the present disclosure may be administered orally or parenterally.

The pharmaceutical composition of the present disclosure may be formulated in a single-dose form or packaged in multi-dose vessels using a pharmaceutically acceptable carrier and/or excipient, according to methods that may be easily carried out by those skilled in the art.

In addition, the present disclosure
relates to probiotics comprising a *Lactococcus lactis* GCWB1176 strain deposited under Accession No. KCCM12687P.

The probiotics may further comprise one or more selected from the five lactic acid bacteria strains isolated in the Examples below, and in addition, may further comprise known strains helpful for the purpose of the present disclosure.

The probiotics may be used at a live bacterial content of 1 × 10¹ to 1 × 10¹³ CFU/g, and the probiotics may be usefully used for enhancing immune function.

In the present disclosure, the dosage of the *Lactococcus lactis* GCWB1176 strain is preferably determined in consideration of the administration method, the age, sex, body weight and severity of the disease of the user, and the like.

As an example, the *Lactococcus lactis* GCWB1176 strain may be administered by dividing the live bacterial content of 1 × 10¹ to 1 × 10¹³ CFU/g per day once or twice or more.

In addition, a food composition, a health functional food composition, a pharmaceutical composition, and probiotics comprising the above ingredients may be administered by dividing the live bacterial content of 1 × 10¹ to 1 × 10¹³ CFU/g per day based on the active ingredient once or twice or more.

However, the above dosage is only an example, and may be changed by a doctor's prescription according to the user's condition.

Hereinafter, the present disclosure will be described in detail by way of the following examples. However, the following examples are only for illustrating the present disclosure, and the scope of the present disclosure is not limited to the following examples.

### [Mode for Invention]

### Example 1: Isolation and identification of Lactococcus lactis GCWB1176

### (1) Isolation of probiotics

The cheeses collected from each region were put into sterile physiological saline in an amount that was 10 times their volume, and homogenized. The homogenized samples were diluted by 10 steps in sterile physiological saline to isolate strains by the dilution plating method. The diluted strain samples were smeared on MRS medium (MRS broth agar; BD Difco), and then anaerobically cultured at 37°C for 72 hours. Colonies that appeared on the MRS agar plate were inoculated a second time on PCA medium (MBcell, South Korea) containing 0.005% BCP, which is a pH indicator, and the colonies in which the purple medium turned yellow were inoculated a third time on the MRS agar plate to purely isolate probiotics.

### (2) Identification of Lactococcus lactis GCWB1176

Chromosomal DNA extraction and purification were performed for the strain purely isolated in (1) above. Two universal primers, 27F (5'-AGAGTTTGATCMTGGCTCAG-3') and 1492R (5'-TACGGYTACCTTGTTACGACTT-3') were used to perform amplification of the 16s rRNA gene, and then sequencing analysis of the amplified 16s rRNA gene was performed. Using the analyzed 16s rRNA sequence data and EzTaxon server (http://www.ezbiocloud.net), only 4 strains corresponding to GRAS (Generally Recognized as Safe) were selected, and the results are as shown in Table 1 below.

**[Table 1]**

| Identified strains |
|---|
| *Pediococcus acidilactici* GCWB1085 |
| *Lactococcus lactis subsp. cremoris* GCWB1177 |
| *Lactococcus lactis* GCWB1176 |
| *Bifidobacterium longum* GCWB 1136 |

The results of 16S rRNA sequencing analysis for *Lactococcus lactis* GCWB1176 were as follows:
**<16S rRNA of bacterial strain, *Lactococcus lactis* GCWB1176>**

CTCAGGACGA ACGCTGGCGG CGTGCCTAAT ACATGCAAGT TGAGCGCTGA
AGGTTGGTAC
TTGTACCAAC TGGATGAGCA GCGAACGGGT GAGTAACGCG TGGGGAATCT
GCCTTTGAGC
GGGGGACAAC TTTGGAAACG AATGCTAATA CCGCATAAAA ACTTTAAACA
CAAGTTTTAA
GTTTGAAAGA TGCAATTGCA TCACTCAAAG ATGATCCCGC GTTGTATTAG
CTAGTTGGTG
AGGTAAAGGC TCACCAAGGC GATGATACAT AGCCGACCTG AGAGGGTGAT
CGGCCACATT
GGGACTGAGA CACGGCCCAA ACTCCTACGG GAGGCAGCAG TAGGGAATCT
TCGGCAATGG
ACGAAAGTCT GACCGAGCAA CGCCGCGTGA GTGAAGAAGG TTTTCGGATC
GTAAAACTCT
GTTGGTAGAG AAGAACGTTG GTGAGAGTGG AAAGCTCATC AAGTGACGGT
AACTACCCAG
AAAGGGACGG CTAACTACGT GCCAGCAGCC GCGGTAATA CGTAGGTCCC
GAGCGTTGTC
CGGATTTATT GGGCGTAAAG CGAGCGCAGG TGGTTTATTA AGTCTGGTGT
AAAAGGCAGT

GGCTCAACCA TTGTATGCAT TGGAAACTGG TAGACTTGAG TGCAGGAGAG
GAGAGTGGAA
TTCCATGTGT AGCGGTGAAA TGCGTAGATA TATGGAGGAA CACCGGTGGC
GAAAGCGGCT
CTCTGGCCTG TAACTGACAC TGAGGCTCGA AAGCGTGGGG AGCAAACAGG
ATTAGATACC
CTGGTAGTCC ACGCCGTAAA CGATGAGTGC TAGATGTAGG GAGCTATAAG
TTCTCTGTAT
CGCAGCTAAC GCAATAAGCA CTCCGCCTGG GGAGTACGAC CGCAAGGTTG
AAACTCAAAG
GAATTGACGG GGGCCCGCAC AAGCGGTGGA GCATGTGGTT TAATTCGAAG
CAACGCGAAG
AACCTTACC AGGTCTTGAC ATACTCGTGC TATTCCTAGA GATAGGAAGT
TCCTTCGGGA
CACGGGATAC AGGTGGTGCA TGGTTGTCGT CAGCTCGTGT CGTGAGATGT
TGGGTTAAGT
CCCGCAACGA GCGCAACCCC TATTGTTAGT TGCCATCATT AAGTTGGGCA
CTCTAACGAG
ACTGCCGGTG ATAAACCGGAG GAAGGTGGGGA TGACGTCAAA TCATCATGCC
CCTTATGACC
TGGGCTACAC ACGTGCTACA ATGGATGGTA CAACGAGTCG CGAGACAGTG
ATGTTTAGCT
AATCTCTTAA AACCATTCTC AGTTCGGATT GTAGGCTGCA ACTCGCCTAC
ATGAAGTCGG
AATCGCTAGT AATCGCGGAT CAGCACGCCG CGGTGAATAC GTTCCCGGGC
CTTGTACACA
CCGCCCGTCA CACCACGGGA GTTGGGAGTA CCCGAAGTAG GTTGCCTAAC
CGCAAGGAGG
GCGCTTCCTA AGGTAAGACC GATGACTGGG GTGAAGTCGT A

### Example 2: Isolation and identification of Bifidobacterium breve GCWB1144

### (1) Isolation of probiotics

The feces of 3-month-old infants were diluted by 10 steps in sterile physiological saline to isolate strain by the dilution plating method. The diluted fecal sample was smeared on BSM agar medium (Bifidus Selective Medium Agar; Sigma, USA), and then anaerobically cultured at 37°C for 72 hours. Colonies that appeared on the BSM agar plate were inoculated a second time on PCA medium (MBcell, South Korea) containing 0.005% BCP, which is a pH indicator, and the colonies in which the purple medium turned yellow were inoculated a third time on BL agar medium (MBcell, South Korea) to purely isolate probiotics.

### (2) Identification of Bifidobacterium breve GCWB1144

Chromosomal DNA extraction and purification were performed on the strain purely isolated in Example 1 above. Two universal primers, 27F (5'-AGAGTTTGATCMTGGCTCAG-3') and 1492R (5'-TACGGYTACCTTGTTACGACTT-3') were used to perform amplification of the 16s rRNA gene, and then sequencing analysis of the amplified 16s rRNA gene was performed. Using the analyzed 16s rRNA sequence data and EzTaxon server (http://www.ezbiocloud.net), only 1 strain corresponding to GRAS (Generally Recognized as Safe) was selected, and the result is as shown in Table 2 below.

**[Table 2]**

| Identified strains |
|---|
| *Bifidobacterium breve* GCWB1144 |

### Experimental Example 1: Evaluation of immune-enhancing efficacy using macrophages

### 1-1) Cytotoxicity evaluation of strains (LDH leakage and CCK-8 assay)

The mouse macrophage line RAW 264.7 cell line was distributed from the Korea Cell Line Bank, and suspended so that the cell concentration was 5 × 10⁵ cells/ml, and 100 µl of each suspension was dispensed into a 96-well plate, and the samples were treated for each concentration as shown in Table 3 below, and then cultured for 24 hours.

Cytotoxicity was measured using a CCK-8 assay kit and a cytotoxicity LDH assay kit, and the results are as shown in Table 3 below.

**[Table 3]**

| | Concentration (µg/ml) | LDH (Fold of control) (24 hr) | MTT ($ of control) (24 hr) |
|---|---|---|---|
| Normal group | - | 1.00±0.02 | 100.0±2.85 |
| Excipient | - | 0.94±0.02 | 104.3±3.32 |
| GCWB1085 | 1 | 0.96±0.03 | 105.3±2.35 |
| | 10 | 0.97±0.01 | 108.9±5.31 |
| | 100 | 0.94±0.03 | 113.1±4.62* |
| | 1000 | 0.56±0.02*** | 102.8±1.68 |
| GCWB1177 | 1 | 1.01±0.01 | 95.9±1.91 |
| | 10 | 0.98±0.04 | 95.6±3.37 |
| | 100 | 0.76±0.01*** | 95.2±2.74 |
| | 1000 | 0.64±0.02*** | 92.112.30 |
| *Lactococcus lactis* GCWB1176 | 1 | 0.96±0.03 | 100.8±2.98 |
| | 10 | 0.98±0.01 | 105.8±4.59 |
| | 100 | 1.00±0.02 | 92.7±4.03 |
| | 1000 | 0.87±0.01** | 63.3±1.86*** |
| GCWB1136 | 1 | 0.97±0.00 | 102.7±4.82 |
| | 10 | 1.03±0.02 | 102.1±7.88 |
| | 100 | 1.03±0.02 | 97.6±4.78 |
| | 1000 | 1.20±0.01 | 93.5±3.83 |
| GCWB1144 | 1 | 1.00±0.01 | 97.5±4.60 |
| | 10 | 0.97±0.02 | 101.9±1.82 |
| | 100 | 0.85±0.01* | 95.0±6.47 |
| | 1000 | 0.96±0.04 | 98.115.99 |

| | | | |
|---|---|---|---|
| (*P < 0.001 vs. normal group; **P < 0.01 vs. normal group; ***P < 0.05 vs. normal group) | | | |

### (1) LDH assay results

The five lactic acid bacteria in Table 3 above were treated in mouse Raw26.7 macrophages at a concentration of 1 to 1,000 µg/ml for 24 hours, and then as a result of performing the LDH assay, all of 5 strains did not show cytotoxicity. However, the LDH value was significantly reduced at concentrations of 100 pg/ml or 1,000 pg/ml of GCWB1085, GCWB1177, GCWB1176, and GCWB1144 strains.

### (2) MTT assay results

The five lactic acid bacteria in Table 3 above were treated in mouse Raw26.7 macrophages at a concentration of 1 to 1,000 µg/ml for 24 hours, and then as a result of performing the MTT assay, all of 5 strains did not show cytotoxicity at concentrations of 100 ug/ml or less. However, cytotoxicity was confirmed at the concentration of 1,000 pg/ml of the GCWB1176 strain.

### (3) Concentration for performing experiments

Subsequent experiments were performed at a concentration of 1 to 100 pg/ml that did not show cytotoxicity in all strains.

### 1-2) Measurement of nitric oxide (NO)

The mouse macrophage line RAW 264.7 cell line was distributed from the Korea Cell Line Bank, and suspended so that the cell concentration was 5 × 10⁵ cells/ml, and 100 µl of each suspension was dispensed into a 96-well plate. These were treated with the samples, 5 strains in Table 3 above, at each concentration of 1 µg/ml and 10 µg/ml for 48 hours (positive control was treated with LPS 10 ng/ml), and then cultured for 24 hours. Thereafter, 50 µL of the culture medium was transferred to a 96-well plate, mixed with Griess reagent I (NED solution) and Griess reagent II (Sulfanilamide solution) in the same amount, and reacted in the dark for 10 minutes, and then, it was measured at 540 nm using a microplate reader within 30 minutes.

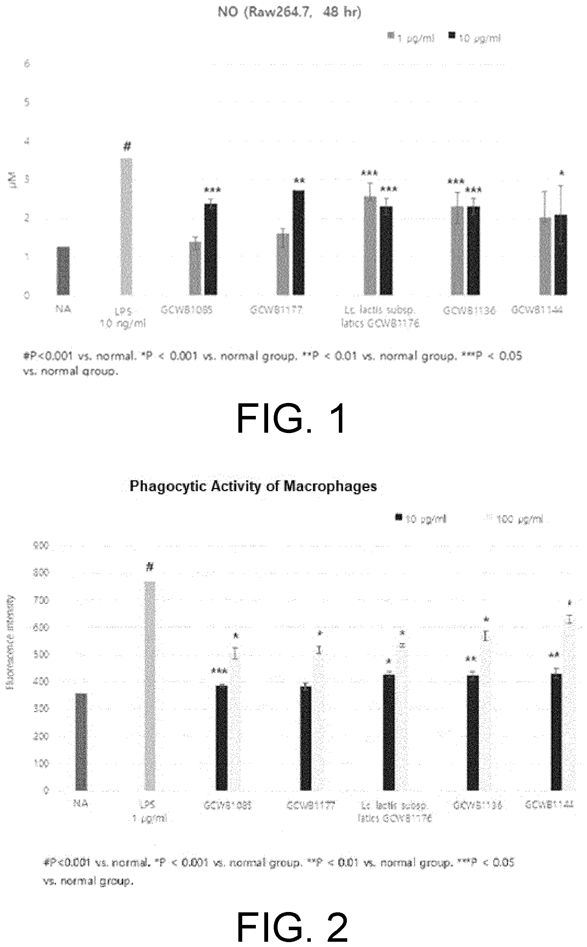

The experimental results are shown graphically in Figure 1. As confirmed from Figure 1, in the case of the GCWB1176 strain of the present disclosure, it was confirmed that the amount of NO produced was significantly increased at both concentrations of 1 µg/ml and 10 pg/ml.

In terms of immune response, immune cells are activated only when there is an initial inflammatory response, and immunity is thereby enhanced, and thus, in contrast to anti-inflammatory, the occurrence of NO rather plays a role in increasing immunity. Therefore, it can be confirmed from the above experiment that the GCWB1176 strain of the present disclosure has an excellent immune function enhancing effect.

### 1-3) Measurement of cytokine secretion

The mouse macrophage line RAW 264.7 cell line was distributed from the Korea Cell Line Bank, and suspended so that the cell concentration was 5 × 10⁵ cells/ml, and 100 µl of each suspension was dispensed into a 96-well plate. These were treated with the samples, 5 strains in Table 3 above, at each concentration of 1 pg/ml, 10 pg/ml and 100 pg/ml (positive control was treated with LPS 10 ng/ml), and then cultured for 3 hours for TNF-alpha, for 30 hours for IFN-gamma, for 30 hours for IL-lbeta, for 20 hours for IL-10, and for 20 hours for IL-12. Thereafter, the amount of cytokines (TNF-alpha, IFN-gamma, IL-lbeta, IL-10, IL-12) secreted from the culture supernatant to the culture medium was measured using an ELISA kit (R&D system). The measured results are as shown in Table 4 below.

**[Table 4]**

| | | Fold | | | | |
|---|---|---|---|---|---|---|
| | Dose (pg/ml) | TNF-alpha | IFN-gamma | IL-1beta | IL-10 | IL-12 |
| Normal group | | 1.00 | 1.00 | 1.00 | 1.00 | 1.00 |
| Excipient (ONLY) | | -0.61 | 1.00 | 1.02 | 1.04 | 1.03 |
| LPS 10 ng/ml + ConA 1 ug/ml | | 2527.61^{#} | 372.69^{#} | 11.01^{#} | 1.91^{#} | 1.44^{#} |
| GCWB1085 | 1 | 1.04 | 10.36* | 1.07 | 0.84 | 1.82** |
| | 10 | 1.06 | 42.69* | 1.27** | 1.09 | 7.88* |
| | 100 | 1.75* | 286.08* | 1.82* | 1.63* | 10.42* |
| GCWB1177 | 1 | 1.01 | 1.26** | 1.01 | 1.04 | 1.08 |
| | 10 | 1.98* | 5.15* | 1.13*** | 1.77* | 1.82* |
| | 100 | 3.96* | 24.57* | 1.66* | 3.77* | 2.12* |
| *Lactococcus lactis* GCWB1176 | 1 | 0.99 | 6.36* | 1.06 | 0.89 | 2.93* |
| | 10 | 1.53* | 67.89* | 1.28*** | 1.13 | 7.70* |
| | 100 | 23.73* | 190.31* | 2.50* | 3.99* | 10.55* |
| GCWB1136 | 1 | 1.00 | 2.28* | 1.09 | 1.17*** | 1.10*** |
| | 10 | 1.09** | 9.28* | 1.07 | 1.76* | 1.73* |
| | 100 | 1.64* | 16.87* | 1.16*** | 3.16* | 2.11* |
| GCWB1144 | 1 | 0.92 | 1.08 | 0.96 | 0.99 | 1.08 |
| | 10 | 1.02 | 10.20* | 1.13** | 1.73* | 1.66* |
| | 100 | 3.14* | 13.32* | 1.21* | 3.70* | 1.80* |

| | | | | | | |
|---|---|---|---|---|---|---|
| (#P<0.001 vs. normal; *P < 0.001 vs. normal group; **P < 0.01 vs. normal group; ***P < 0.05 vs. normal group) | | | | | | |

### (1) Measured results of TNF-alpha

In the case of the GCWB1176 strain of the present disclosure, it was confirmed that the amount of TNF-α was significantly increased at a concentration of 10 pg/ml or more.

### (2) Measured results of IFN-gamma

In the case of the GCWB1176 strain of the present disclosure, it was confirmed that the amount of IFN-γ was significantly increased at concentrations of 1, 10 and 100 pg/ml or more.

### (3) Measured results of IL-1beta

In the case of the GCWB1176 strain of the present disclosure, it was confirmed that the amount of IL-1β was significantly increased at a concentration of 10 pg/ml or more.

### (4) Measured results of IL-10

In the case of the GCWB1176 strain of the present disclosure, it was confirmed that the amount of IL-10 was significantly increased at a concentration of 100 pg/ml or more.

### (5) Measured results of IL-12

In the case of the GCWB1176 strain of the present disclosure, it was confirmed that the amount of IL-12 was very remarkably increased at all concentrations of 1, 10 and 100 pg/ml.

### (6) Experimental results of cytokine secretion

As confirmed from the above data, it was confirmed that the GCWB1176 strain of the present disclosure significantly increased cytokine secretion in the macrophage line of ICR mouse.

### 1-4) Measurement of phagocytic activity of macrophages

The mouse macrophage line RAW 264.7 cell line was distributed from the Korea Cell Line Bank, and suspended so that the cell concentration was 5 × 10⁵ cells/ml, and 100 µl of each suspension was dispensed into a 96-well plate. These were treated with the samples, 5 strains in Table 3 above, at each concentration of 10 µg/ml and 100 ug/ml for 24 hours (positive control was treated with LPS 10 ng/ml), and then treated with 100 µl of fluorescein-5-isothiocyanate (FITC)-labeled *E. coli* (Molecular Probe, Eugene, OR, U.S.A.) for 2 hours. Thereafter, the non-phagocytized cells remaining in the supernatant were removed by washing, and the phagocytized *E. coli* was measured by analyzing FITC with a fluorescence analyzer. In addition, cell viability was measured by CCK-8 assay to correct macrophage activity.

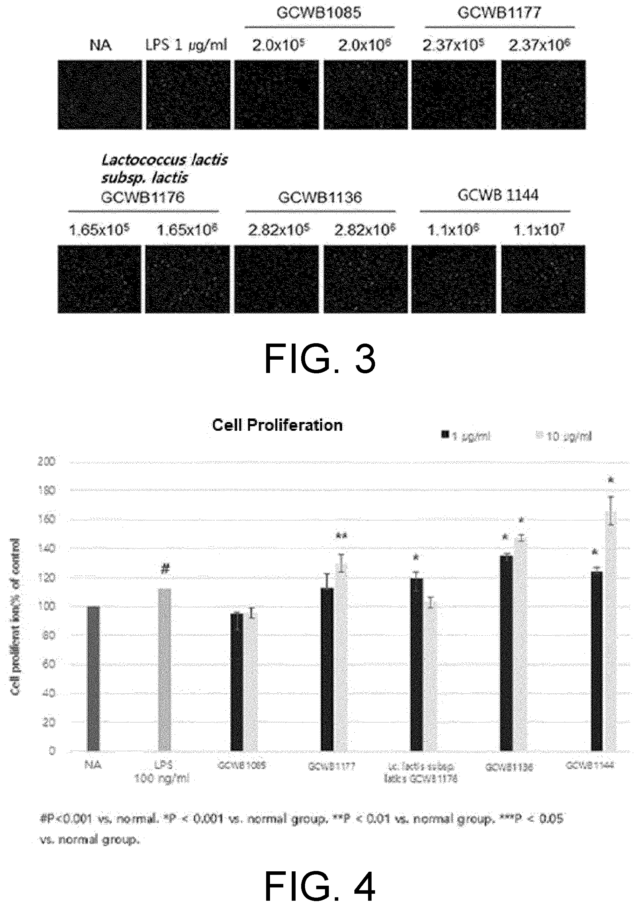

The experimental results are shown as in Figures 2 and 3. As confirmed from Figures 2 and 3, in the case of the GCWB1176 strain of the present disclosure, it was confirmed that the phagocytic activity was significantly increased at both concentrations of 10 µg/ml and 100 pg/ml.

### Experimental Example 2: Evaluation of immune-enhancing efficacy using splenocytes of ICR mouse

### 2-1) Measurement of splenocyte proliferation in ICR mice

In order to isolate splenocytes, the spleen was aseptically extracted from ICR mice, and then washed with RPMI 1640 solution, and crushed. The isolated cell suspension was passed through a 200 mesh stainless steel sieve, and then centrifuged at 4°C and 1,200 rpm for 3 minutes. The cell pellets were suspended in ACK buffer for 5 minutes to remove red blood cells, and the leaked splenocytes were suspended in RPMI 1640 containing 10% fetal bovine serum and 1% penicillin-streptomycin so that the cell concentration is 1 × 10⁵ cells/ml, and 200 µl of each suspension was dispensed into a 96-well plate.

These were treated with the samples, 5 strains in Table 3 above, at each concentration of 1 µg/ml and 10 µg/ml for 72 hours (positive control was treated with LPS 100 ng/ml), and then cultured for 48 hours, and the proliferation of splenocytes was measured using a CCK-8 assay kit, and the results are as shown in Table 5 below and Figure 4.

**[Table 5]**

| | Dose (pg/ml) | WST-1 (72 hr) |
|---|---|---|
| Normal group | - | 100.0±1.2 |
| LPS 100 ng/ml | - | 112.211.5# |
| GCWB 1085 | 1 | 95.111.3 |
| | 10 | 98.6±3.5 |
| GCWB 1177 | 1 | 113.119.3 |
| | 10 | 129.816.2** |
| *Lactococcus lactis* GCWB1176 | 1 | 119.913.6* |
| | 10 | 102.913.7 |
| GCWB1136 | 1 | 135.011.8* |
| | 10 | 147.512.2* |
| GCWB1144 | 1 | 124.212.8* |
| | 10 | 166.019.6* |

| | | |
|---|---|---|
| (#P<0.001 vs. normal; *P < 0.001 vs. normal group; **P < 0.01 vs. normal group; ***P < 0.05 vs. normal group) | | |

As confirmed from Table 5 above and Figure 4, in the case of the GCWB1176 strain of the present disclosure, it was confirmed that the splenocyte proliferation rate was significantly increased at a concentration of 10 pg/ml.

### 2-2) Measurement of nitric oxide (NO)

The splenocytes were suspended so that the concentration was 5 × 10⁵ cells/ml, and 100 µl of each suspension was dispensed into a 96-well plate. These were treated with the samples, 5 strains in Table 3 above, at each concentration of 1 µg/ml and 10 µg/ml for 48 hours (positive control was treated with LPS 10 ng/ml), and then cultured for 24 hours. Thereafter, 50 µL of the culture medium was transferred to a 96-well plate, mixed with Griess reagent I (NED solution) and Griess reagent II (Sulfanilamide solution) in the same amount, and reacted in the dark for 10 minutes, and then, it was measured at 540 nm using a microplate reader within 30 minutes.

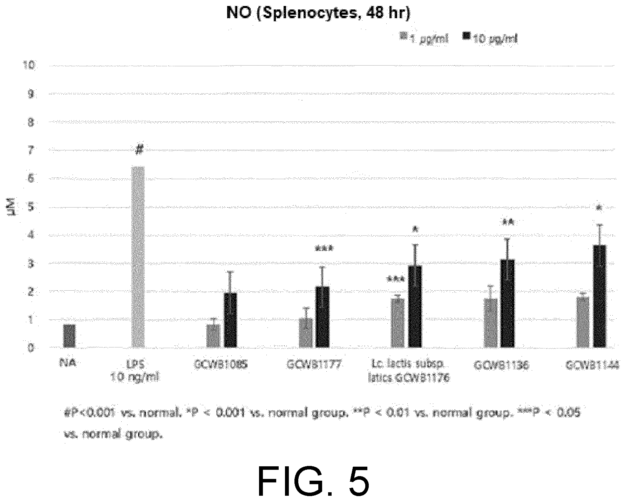

The experimental results are shown graphically in Figure 5. As confirmed from Figure 5, in the case of the GCWB1176 strain of the present disclosure, it was confirmed that the amount of NO produced was significantly increased at both concentrations of 1 µg/ml and 10 pg/ml.

### 2-3) Measurement of cytokine secretion

The splenocytes were suspended so that the concentration was 1 × 10⁵ cells/ml, and 200 µl of each suspension was dispensed into a 96-well plate.

These were treated with the samples, 5 strains in Table 3 above, at each concentration of 1 pg/ml, 10 pg/ml and 100 pg/ml (positive control was treated with LPS 10 ng/ml and ConA 1 ug/ml), and then cultured for 3 hours for TNF-alpha, for 30 hours for IFN-gamma, for 30 hours for IL-1beta, for 20 hours for IL-10, and for 20 hours for IL-12. Thereafter, the amount of cytokines (TNF-alpha, IFN-gamma, IL-1beta, IL-10, IL-12) secreted from the culture supernatant to the culture medium was measured using an ELISA kit (R&D system). The measured results are as shown in Table 6 below.

**[Table 6]**

| | | Fold | | | | |
|---|---|---|---|---|---|---|
| Classification | Dose (pg/ml) | TNF-alpha | IFN-gamma | IL-1beta | IL-10 | IL-12 |
| Normal group | - | 1.00 | 1.00 | 1.00 | 1.00 | 1 |
| Excipient (ONLY) | - | 1.00 | 0.97 | 1.03 | 0.93 | 0.99 |
| LPS 10ng/ml | - | 567.56 # | 177177.4 8# | 8.80# | 3.67# | 1.77# |
| GCWB1085 | 1 | 1.02 | 31.46* | 1.02 | 1.23** | 2.71* |
| | 10 | 0.99 | 17843.10 * | 1.27** | 2.30* | 29.24* |
| | 100 | 10.90* | 45424.15 * | 1.95* | 7.71* | 46.40* |
| GCWB1177 | 1 | 0.94 | 1.08 | 1.02 | 0.77 | 1.11 |
| | 10 | 2.17* | 27.19* | 1.17** | 1.59* | 2.46* |
| | 100 | 15.17* | 678.35* | 2.08* | 3.88* | 3.06* |
| *Lactococcus lactis* GCWB1176 | 1 | 24.26* | 1100.77* * | 1.06 | 1.57* | 4.33* |
| | 10 | 473.40 * | 47671.44 * | 1.40** | 5.13* | 27.32* |
| | 100 | 195.33 * | 63751.74 *** | 2.90* | 11.66* | 39.86* |
| GCWB1136 | 1 | 1.03 | 4.32* | 1.07 | 1.15** | 1.15* |
| | 10 | 1.36* | 105.81* | 1.19** | 1.87* | 2.24* |
| | 100 | 2.97* | 268.91* | 1.30* | 3.41* | 2.88* |
| GCWB1144 | 1 | 0.94 | 1.03 | 0.97 | 1.04 | 1.06 |
| | 10 | 1.18* | 102.20* | 1.27* | 1.82* | 2.19* |
| | 100 | 8.94* | 254.19* | 1.39* | 3.97* | 2.46* |

| | | | | | | |
|---|---|---|---|---|---|---|
| (#P<0.001 vs. normal; *P < 0.001 vs. normal group; **P < 0.01 vs. normal group; ***P < 0.05 vs. normal group) | | | | | | |

### (1) Measured results of TNF-alpha

In the case of the GCWB1176 strain of the present disclosure, it was confirmed that the amount of TNF-alpha was very remarkably increased at all concentrations of 1, 10 and 100 pg/ml.

### (2) Measured results of IFN-gamma

In the case of the GCWB1176 strain of the present disclosure, it was confirmed that the amount of IFN-gamma was very remarkably increased at all concentrations of 1, 10 and 100 pg/ml.

### (3) Measured results of IL-1beta

In the case of the GCWB1176 strain of the present disclosure, it was confirmed that the amount of IL-1beta was remarkably increased at a concentration of 10 pg/ml or more.

### (4) Measured results of IL-10

In the case of the GCWB1176 strain of the present disclosure, it was confirmed that the amount of IL-10 was remarkably increased at all concentrations of 1, 10 and 100 pg/ml.

### (5) Measured results of IL-12

In the case of the GCWB1176 strain of the present disclosure, it was confirmed that the amount of IL-12 was very remarkably increased at all concentrations of 1, 10 and 100 pg/ml.

### (6) Experimental results of cytokine secretion

As confirmed from the above data, in the case of the GCWB1176 strain of the present disclosure, it was confirmed that the cytokine secretion was remarkably excellent compared to that of the normal group and other strains.

### Experimental Example 3: Measurement of immune-enhancing efficacy of samples in the immunosuppressive agent cyclophosphamide-induced immunosuppressed animal model (in vivo assay)

### 3-1) Experimental method

A 6-week-old SPF ICR mouse (weight: 20 ± 2 g) was purchased from Samtako Bio Korea, Co., Ltd., and intraperitoneally administered the immunosuppressive agent cyclophosphamide at a concentration of 80 mg/kg for 3 days (once a day), and then orally administered the sample GCWB1176 strain at each concentration as shown in Table 7 below once a day for 16 days.

**[Table 7]**

| No. | Test group (n=8) | Concentration |
|---|---|---|
| 1 | Normal control | - |
| 2 | Cyclophosphamide (CTX) | 150 mg/kg |
| 3 | CTX + GCWB1176-L | 1 × 10⁷ CFU/ml |
| 4 | CTX + GCWB1176-H | 1 × 10⁹ CFU/ml |

### 3-2) Measurement of changes in body weight, spleen weight and thymus weight

Changes in body weight were observed by measuring the body weight of the mouse on days 5, 10, and 17, respectively, and the spleen and thymus weights were measured on the last autopsy day, wherein the mouse was observed every 4 days for 24 days after administration of cyclophosphamide and the spleen and thymus weights were measured after the test was completed.

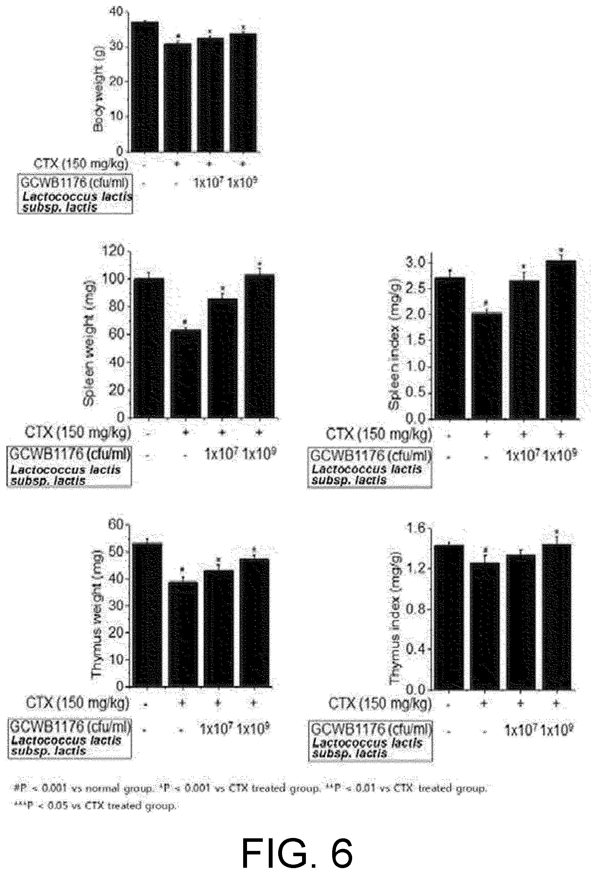

The measured results are as shown in Figure 6.

As confirmed from Figure 6, in the cyclophosphamide (CTX)-administered group, body weight, spleen weight, and thymus weight were significantly decreased compared to those of the normal group. On the other hand, in the CTX + low concentration (1 × 10⁷ CFU/ml) of GCWB1176 strain-administered group and the CTX + high concentration (1 × 10⁹ CFU/ml) of GCWB1176 strain-administered group, body weight, spleen weight and thymus weight were significantly increased compared to those of the cyclophosphamide (CTX) alone-administered group.

### 3-3) Measurement of cytokine secretion

After completion of the experiment in 3-1) above, serum was obtained and stored at -80°C. The amount of cytokines (TNF-alpha, IFN-gamma, IL-4, IL-10, IL-2, IL-12) in serum was measured using an ELISA kit (R&D system, USA).

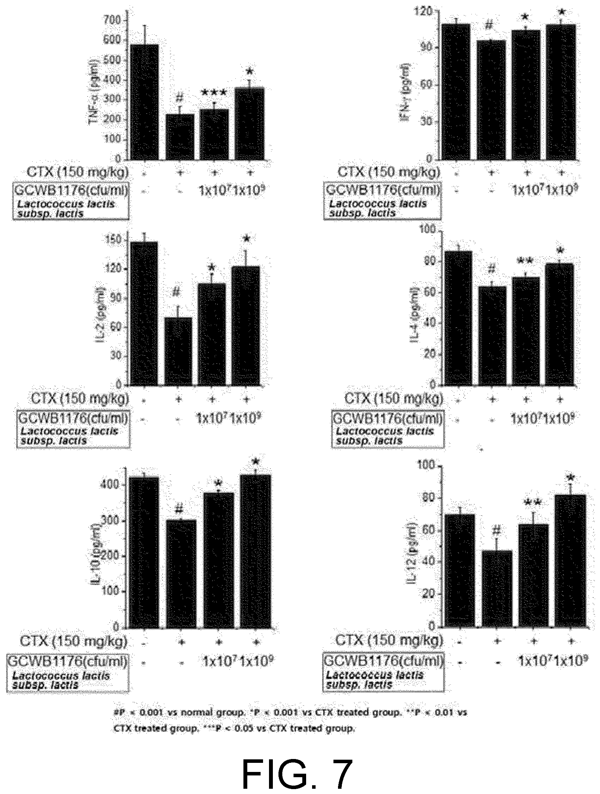

The measured results are as shown in Figure 7.

As confirmed from Figure 7, in the cyclophosphamide (CTX)-administered group, the amounts of TNF-alpha, IFN-gamma, IL-4, IL-10, IL-2, and IL-12 were significantly decreased compared to those of the normal group. On the other hand, in the CTX + low concentration (1 × 10⁷ CFU/ml) of GCWB1176 strain-administered group and the CTX + high concentration (1 × 10⁹ CFU/ml) of GCWB1176 strain-administered group, it was confirmed that the amounts of TNF-alpha, IFN-gamma, IL-4, IL-10, IL-2, and IL-12 were significantly increased compared to those of the cyclophosphamide (CTX) alone-administered group.

### 3-4) Measurement of NK cell activity and cell proliferation rate

NK cell activity was measured using a CCK-8 assay kit. After completion of the experiment in 3-1) above, the spleen was extracted, and splenocytes (1 × 10⁶ cells/mL) isolated from the spleen were separated and cultured in 100 µl in a 96-well plate. These were treated with T-cell mitogen conA (2 pg/ml), and then mixed with 100 ul of the target cells YAC-1 cells (1 × 10⁴ cells/mL), and cultured in a cell incubator for 20 hours. After culture, CCK-8 was added thereto and reacted at 37°C for 4 hours. Absorbance was measured at 450 nm using a microplate reader.

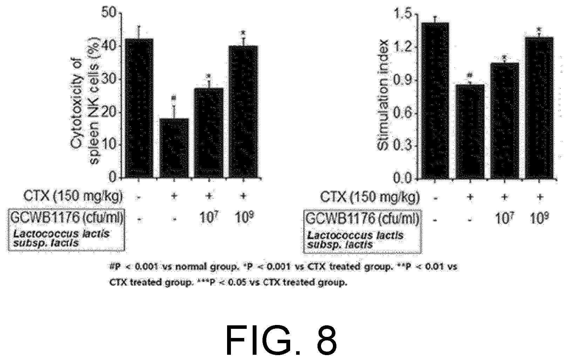

The measured results are as shown in Figure 8.

As confirmed from Figure 8, in the cyclophosphamide (CTX)-administered group, the NK cell activity and the degree of cell proliferation were significantly decreased compared to those of the normal group. On the other hand, in the CTX + low concentration (1 × 10⁷ CFU/ml) of GCWB1176 strain-administered group and the CTX + high concentration (1 × 10⁹ CFU/ml) of GCWB1176 strain-administered group, the NK cell activity and cell proliferation were significantly increased compared to those of the cyclophosphamide (CTX) alone-administered group.

### [Accession Number]

Name of depository institution: Korean Culture Center of Microorganisms (Overseas)
Accession number: KCCM12687P
Accession date: 20200331

## Claims

1. A *Lactococcus lactis* GCWB1176 strain deposited under Accession No. KCCM12687P.

2. A food composition for enhancing immune function, comprising a *Lactococcus lactis* GCWB1176 strain deposited under Accession No. KCCM12687P, one selected from a spray-dried product, a freeze-dried product, a vacuum-dried product, a drum-dried product or a crushed product of the strain, or any one of a culture of the strain, a concentrate, a paste and a dilution of the culture.

3. A health functional food composition for enhancing immune function, comprising a *Lactococcus lactis* GCWB1176 strain deposited under Accession No. KCCM12687P, one selected from a spray-dried product, a freeze-dried product, a vacuum-dried product, a drum-dried product or a crushed product of the strain, or any one of a culture of the strain, a concentrate, a paste and a dilution of the culture.

4. The health functional food composition for enhancing immune function according to claim 3, wherein the health functional food composition further comprises one or more selected from the group consisting of proteins, carbohydrates, fats, nutrients, seasonings, sweeteners, and flavoring agents.

5. The health functional food composition for enhancing immune function according to claim 3, wherein the health functional food composition is any one selected from granules, lemonades, powders, syrups, liquids and solutions, extracts, elixirs, fluid extracts, suspensions, decoctions, infusions, tablets, spirits, capsules, troches, pills, and soft or hard gelatin capsules.

6. Probiotics comprising a *Lactococcus lactis* GCWB1176 strain deposited under Accession No. KCCM12687P.

7. The probiotics according to claim 6, wherein the probiotics have a live bacterial content of 1 × 10¹ to 1 × 10¹³ CFU/g.

8. The probiotics according to claim 6, wherein the probiotics are used for enhancing immune function.
